(19) Europäisches Patentamt
European Patent Office
Office européen des brevets

(11) **EP 3 659 574 A1**

(12) **EUROPÄISCHE PATENTANMELDUNG**

(43) Veröffentlichungstag:
**03.06.2020   Patentblatt 2020/23**

(51) Int Cl.:
*A61K 6/02* (2006.01)          *A61C 13/00* (2006.01)

(21) Anmeldenummer: **18209272.6**

(22) Anmeldetag: **29.11.2018**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Ivoclar Vivadent AG**
**9494 Schaan (LI)**

(72) Erfinder:
• **Jiang, Bo**
  **9470 Werdenberg (CH)**
• **Ritzberger, Christian**
  **9472 Grabs (CH)**
• **Rothbrust, Frank**
  **6822 Röns (AT)**

(74) Vertreter: **Uexküll & Stolberg**
**Partnerschaft von**
**Patent- und Rechtsanwälten mbB**
**Beselerstraße 4**
**22607 Hamburg (DE)**

(54) **VERFAHREN ZUR HERSTELLUNG EINES ZIRKONOXID-ROHLINGS**

(57)    Die Erfindung betrifft ein Verfahren zur Herstellung eines Zirkonoxid-Rohlings durch Einbringen einer Zirkonoxid-Suspension in eine poröse Form und Entformung des gebildeten Rohlings sowie die Verwendung des gebildeten, optional vorgesinterten, Rohlings zur Herstellung einer Dentalrestauration unter Benutzung eines sehr kurzen Dichtsinterprozesses.

EP 3 659 574 A1

**Beschreibung**

Die vorliegende Erfindung betrifft ein Verfahren zur Herstellung eines Zirkonoxid-Rohlings, der nach gewünschter Formgebung innerhalb sehr kurzer Zeit zu dentalen Restaurationen mit ausgezeichneten optischen Eigenschaften dichtgesintert werden kann.

Zur Herstellung vollanatomischer Dentalrestaurationen werden häufig Zirkonoxidkeramiken eingesetzt. Diese bieten eine hohe klinische Sicherheit, sind metallfrei, können auch bei minimal-invasiven Präparationen eingesetzt werden und sind preislich im Vergleich zu anderen metallfreien Restaurationen sehr attraktiv. Üblicherweise werden die Restaurationen aus vorgesinterten Rohlingen herausgefräst oder geschliffen, durch thermische Behandlung dichtgesintert und schließlich gegebenenfalls mit einer Verblendung versehen und/oder glasiert.

Verfahren zur Herstellung von Zirkonoxid-Rohlingen und deren Verarbeitung zu dentalen Restaurationen sind bekannt.

Die WO 2014/209626 beschreibt ein Verfahren zur Herstellung von Zirkonoxid-Rohlingen, die nach Vorsinterung und maschineller Bearbeitung mittels CAD/CAM-Verfahren durch Dichtsintern bei Temperaturen von weniger als 1200°C zu dentalen Restaurationen verarbeitet werden können. Die Restaurationen zeichnen sich dadurch aus, dass sie nicht nur transluzent, sondern auch opaleszent sind. Zur Herstellung der Rohlinge werden Suspensionen von nanoskaligen Zirkonoxid-Teilchen mit einer mittleren Primärpartikelgröße von nicht mehr als 20 nm zum Beispiel in entsprechende Formen gegossen. Die Herstellung der Suspensionen ist jedoch aufwendig, da die als Rohstoff eingesetzten kommerziell erhältlichen Suspensionen erst eines langwierigen Schrittes der Aufkonzentration bedürfen. Die durch das Gießen erhaltenen Grünlinge müssen zudem sehr langsam getrocknet werden, was gerade bei Rohlingen mit einer größeren Dicke zur Vermeidung der Bildung von Rissen mehrere Tage dauert. Schließlich ist auch der gesamte Dichtsintervorgang sehr zeitaufwendig und dauert mehr als 4 Stunden, womit der Wunsch des Patienten nach einer schnellen Fertigstellung der aus dem Rohling geschliffenen und gefrästen Restauration nicht erfüllt werden kann.

Die WO 2009/061410 und die WO 2013/181018 beschreiben Verfahren zur Herstellung von Zirkonoxid-Rohlingen durch Schlickergießen von Suspensionen. Die Rohlinge sollen nach Trocknung, Vorsinterung, maschineller Bearbeitung, z.B. durch CAD/CAM-Verfahren, und anschließendem Dichtsintern transluzente Dentalrestaurationen ergeben. Es werden allerdings weder konkrete Beispiele für das Verfahren und die dabei eingesetzte Suspension noch die genauen Bedingungen für das Dichtsintern und insbesondere dessen Dauer angegeben.

Die EP 826 642 beschreibt ein Verfahren zur Herstellung eines keramischen Gerüstes für eine dentale Restauration. Das Verfahren beinhaltet das Schlickergießen einer Suspension mit Gehalt an Aluminiumoxid- und Zirkonoxid-Teilchen zu einer keramischen Folie, Aufschichten der Folie auf ein Gipsmodell eines Zahnes, Ausüben von Druck zum Verbinden der Folie mit dem Modell, Wärmebehandlung bei 500°C, Sintern bei 1150°C, Beschichten des gesinterten Körpers mit Glaspulver und weiteres Erhitzen.

Die KR 101416654 B1 beschreibt ein Verfahren zur Herstellung von $ZrO_2$-Blöcken unter Verwendung von Zirkonoxid-Abfällen, wie sie insbesondere im Dentallabor anfallen. Aus diesen Abfällen werden nach Entbindern und Mahlen wässrige Zirkonoxid-Suspensionen erzeugt, aus denen durch Schlickerguß Körper mit der gewünschten Form hergestellt werden. Diese Körper werden kalt-isostatisch gepresst und anschließend zweimal gesintert.

Die WO 2018/049331 beschreibt dentale Zirkonoxid-Rohlinge für CAD/CAM-Anwendungen, welche aufgrund der Verwendung unterschiedlicher $ZrO_2$-Teilchen einen Verlauf von Eigenschaften und insbesondere einen Farbverlauf zeigen. Die Rohlinge können durch Schlickergießen von Gemischen von Suspensionen mit unterschiedlich gefärbten und unterschiedlich großen Zirkonoxid-Teilchen gebildet werden, wobei das unterschiedliche Absetzverhalten der Teilchen ausgenutzt werden soll.

Die konventionellen Verfahren zur Herstellung von Zirkonoxid-Rohlingen sind jedoch aufwendig und die mit ihnen erzeugten Rohlinge benötigen für die Dichtsinterung lange Prozesszeiten, insbesondere um die gewünschten optischen Eigenschaften, wie eine hohe Transluzenz, zu erzielen. Typischerweise dauert der gesamte Dichtsinterprozeß von konventionellen Rohlingen beginnend mit dem Aufheizen auf die Sintertemperatur und endend mit der Abkühlung auf Raumtemperatur deutlich mehr als 4 Stunden, was erheblich zu einer unbefriedigend langen Dauer für die Verarbeitung zu Dentalrestaurationen beiträgt.

Der Erfindung liegt daher die Aufgabe zugrunde, ein Verfahren zur Herstellung eines Zirkonoxid-Rohlings bereitzustellen, welches die Nachteile der konventionellen Verfahren vermeidet und die Erzeugung von Rohlingen ermöglicht, die in kurzer Zeit zu dentalen Restaurationen mit sehr guten optischen und mechanischen Eigenschaften verarbeitet werden können.

Diese Aufgabe wird erfindungsgemäß durch das Verfahren zur Herstellung eines Zirkonoxid-Rohlings nach den Ansprüchen 1 bis 16 gelöst. Gegenstand der Erfindung ist auch der Zirkonoxid-Rohling nach den Ansprüchen 17 bis 19, die Verwendung des Zirkonoxid-Rohlings nach Anspruch 20 sowie das Verfahren zur Herstellung einer dentalen Restauration nach den Ansprüchen 21 bis 24.

Das erfindungsgemäße Verfahren zur Herstellung eines Zirkonoxid-Rohlings zeichnet sich dadurch aus, dass

(a) eine Suspension von Zirkonoxid in einem flüssigen Medium in eine Poren aufweisende Form eingebracht,
(b) das flüssige Medium zumindest teilweise über die Poren entfernt und
(c) der gebildete Rohling aus der Form entfernt wird,

wobei die Suspension einen Gehalt an Zirkonoxid von 68 bis 88 Gew.-%, bevorzugt 70 bis 86 Gew.-% und besonders bevorzugt 75 bis 85 Gew.-% aufweist.

Es wurde überraschend gefunden, dass ein mittels des erfindungsgemäßen Verfahrens erhältlicher Rohling nach entsprechender Formgebung in einem nur sehr kurzen Zeitraum zu dentalen Restaurationen dichtgesintert werden kann, die dennoch sehr gute optische Eigenschaften und insbesondere eine hohe Transluzenz aufweisen und damit auch in ästhetischer Hinsicht die hohen Anforderungen an dentale Restaurationen erfüllen. Damit ist es möglich, für einen Patienten innerhalb nur einer Sitzung beim Zahnarzt eine sowohl gewünscht geformte als auch dichtgesinterte Dentalrestauration aus einem Rohling zu erzeugen und diese bei ihm einzusetzen. Eine derartig schnelle Versorgung mit einer Dentalrestauration wird auch als "chairside"-Behandlung bezeichnet und sie ist für den Patienten naturgemäß überaus attraktiv. Damit ist das erfindungsgemäße Verfahren konventionellen Verfahren deutlich überlegen, bei denen gerade zur Erzielung einer zufriedenstellenden Transluzenz sehr lange Sinterzeiten in Kauf genommen werden müssen.

Das Zirkonoxid in der Suspension hat insbesondere eine Teilchengröße von 50 bis 250 nm, bevorzugt von 60 bis 250 nm und besonders bevorzugt 80 bis 250 nm, gemessen als $d_{50}$-Wert, bezogen auf das Volumen der Teilchen. Die Bestimmung der Teilchengröße erfolgt insbesondere mit dem Verfahren der statischen Laserbeugung (SLS) nach ISO 13320:2009, z.B. unter Benutzung eines Partikelanalysators LA-960 der Firma Horiba, oder der dynamischen Lichtstreuung (DLS) nach ISO 22412:2017, z.B. unter Benutzung eines Partikelmessgeräts Nano-flex der Firma Colloid Metrix.

Die Primärpartikelgrösse des Zirkonoxids liegt insbesondere im Bereich von 30 bis 100 nm und sie wird üblicher Weise ebenfalls mit einem wie vorstehend beschriebenen Verfahren der dynamischen Lichtstreuung (DLS) oder mittels Rasterelektronenmikroskopie bestimmt.

Bei dem Zirkonoxid handelt es sich insbesondere um Zirkonoxid auf Basis von polykristallinem tetragonalem Zirkonoxid (TZP). Bevorzugt ist Zirkonoxid, das mit $Y_2O_3$, $La_2O_3$, $CeO_2$, $MgO$ und/oder $CaO$ stabilisiert ist und insbesondere mit 2 bis 14 Mol-%, vorzugsweise mit 2 bis 10 Mol-% und besonders bevorzugt 2 bis 8 Mol-% dieser Oxide, bezogen auf den Gehalt an Zirkonoxid, stabilisiert ist.

Das im erfindungsgemäßen Verfahren eingesetzte Zirkonoxid kann auch eingefärbt sein. Die gewünschte Einfärbung wird dabei insbesondere erzielt, indem das Zirkonoxid mit einem oder mehreren färbenden Elementen versetzt wird. Die Zugabe von färbenden Elementen wird bisweilen auch als Dotierung bezeichnet und sie erfolgt üblicherweise während der Herstellung des Zirkonoxidpulvers durch Cofällung und anschließende Kalzinierung. Beispiele für geeignete färbende Elemente sind Fe, Mn, Cr, Ni, Co, Pr, Ce, Eu, Gd, Nd, Yb, Tb, Er und Bi.

Bei dem Zirkonoxid in der Suspension kann es sich auch um ein Gemisch von Zirkonoxidpulvern mit unterschiedlicher Zusammensetzung handeln, die insbesondere zu einer unterschiedlichen Färbung und/oder Transluzenz bei der schließlich erzeugten dentalen Restauration führt. Mithilfe eines Gemisches aus unterschiedlich gefärbten Zirkonoxidpulvern kann somit die gewünschte Farbe für die aus dem Rohling erzeugte dentale Restauration einfach und in gezielter Weise eingestellt werden. In gleicher Weise kann auch die Transluzenz der erzeugten dentalen Restauration durch Verwendung eines Gemisches von Zirkonoxidpulvern unterschiedlicher Transluzenz gezielt eingestellt werden. Der Grad der Transluzenz der erzeugten dentalen Restauration kann insbesondere durch den Gehalt an Yttriumoxid der verwendeten Zirkonoxidpulver gesteuert werden.

Bei der Suspension kann es sich auch um ein Gemisch von unterschiedlichen Suspensionen zum Beispiel mit unterschiedlich gefärbtem Zirkonoxid handeln.

Im erfindungsgemäßen Verfahren liegt das Zirkonoxid als Suspension in einem flüssigen Medium vor. Dieses flüssige Medium enthält insbesondere Wasser.

Es ist darüber hinaus bevorzugt, dass das flüssige Medium lediglich geringe Mengen an organischen Komponenten aufweist und es enthält daher organische Komponenten in einer Menge von insbesondere nicht mehr als 5 Gew.-%, bevorzugt nicht mehr als 3 Gew.-%, weiter bevorzugt nicht mehr als 2 Gew.-% und besonders bevorzugt nicht mehr als 1 Gew.-%, bezogen auf die Menge an Feststoff in der Suspension.

In einer weiteren bevorzugten Ausführungsform enthält das flüssige Medium organische Komponenten in einer Menge von 0,05 bis 5 Gew.-%, insbesondere 0,1 bis 3 Gew.-%, besonders bevorzugt 0,1 bis 2 Gew.-% und besonders bevorzugt 0,1 bis 1 Gew.-%, bezogen auf die Menge an Feststoff in der Suspension.

Als organische Komponenten kommen insbesondere Dispergiermittel, Bindemittel, Mittel zur Einstellung des pH-Wertes, Stabilisierungsmittel und/oder Entschäumer infrage.

Das Dispergiermittel dient dazu, die Agglomeration von suspendierten Teilchen zu größeren Teilchen zu vermeiden. Die Menge an Dispergiermittel in dem flüssigen Medium beträgt insbesondere 0,01 bis 5 Gew.-%, bevorzugt 0,1 bis 2 Gew.-% und besonders bevorzugt 0,1 bis 1 % Gew.-%, bezogen auf die Menge an Feststoff in der Suspension.

Geeignete Dispergiermittel sind wasserlösliche Polymere, wie Polyvinylalkohole, Polyethylenimine, Polyacrylamide, Polyethylenoxide, Polyethylenglykole, Homo- und Copolymere der (Meth)acrylsäure, Polyvinylpyrrolidon, Biopolymere, wie Stärke, Alginate, Gelatine, Celluloseether, wie Carboxymethylcellulose, Vinylsulfonsäure- und Vinylphosphonsäure.

Bevorzugte Dispergiermittel sind Aminoalkohole, wie Ethanolamin, Glykole, wie Ethylenglykol und Dipropylenglykol, Carbonsäuren, wie Maleinsäure und Citronensäure, und Carbonsäuresalze, sowie Mischungen dieser Dispergiermittel.

Es ist weiter bevorzugt, dass das flüssige Medium mindestens eine Verbindung ausgewählt aus Aminoalkohol, Glykol, Carbonsäure und Carbonsäuresalz enthält. Besonders bevorzugt enthält das flüssige Medium mindestens eine Verbindung ausgewählt aus Ethanolamin, Ethylenglykol, Dipropylenglykol, Citronensäure und Citronensäuresalz.

Das Bindemittel fördert den Zusammenhalt von Teilchen in dem nach Schritt (c) vorliegenden Rohling. Die Menge an Bindemittel in dem flüssigen Medium beträgt insbesondere 0,01 bis 5 Gew.-%, bevorzugt 0,01 bis 3 Gew.- und besonders bevorzugt 0,01 bis 2 Gew.-%, bezogen auf die Menge an Feststoff in der Suspension.

Beispiele für geeignete Bindemittel sind Methylcellulose, Natriumcarboxymethylcellulose, Stärken, Dextrine, Natriumalginat, Ammoniumalginat, Polyethylenglykole, Polyvinylbutyral, Acrylatpolymere, Polyethylenimin, Polyvinylalkohol und Polyvinylpyrrolidon.

Bevorzugte Bindemittel sind Polyvinylalkohol, Polyvinylacetat, Polyvinylpyrrolidon, Polyacrylsäure, Copolymere von Acrylsäureester und Acrylsäure, Polyethylacrylat, Polymethacrylsäure, Polymethylmethacrylat, Ammoniumpolyacrylat, Ammoniumpolymethacrylat, Polyethylenglykol und feste Copolymere aus Ethylenglykol und Propylenglykol.

Es ist ein weiterer Vorteil des durch das erfindungsgemäße Verfahren erhältlichen Rohlings, dass er trotz geringer Anteile an Bindemittel auch ohne vorherige Vorsinterung über eine ausreichende Festigkeit verfügt, um durch maschinelle Bearbeitung, z.B. mittels Schleifen und Fräsen, zur gewünschten dentalen Restauration verarbeitet werden zu können.

Als Mittel zur Einstellung des pH-Wertes und als Stabilisierungsmittel kommen insbesondere Säuren und Basen infrage, wie Carbonsäuren, z.B. 2-(2-Methoxyethoxy)essigsäure und 2-[2-(2-Methoxyethoxy)ethoxy]essigsäure, anorganische Säuren, z.B. Salzsäure und Salpetersäure, sowie Ammoniumhydroxid und Tetramethylammoniumhydroxid. Es ist bevorzugt, dass das flüssige Medium Tetramethylammoniumhydroxid enthält.

Der Entschäumer dient der Vermeidung von Luftblasen in der Suspension. Er wird typischerweise in einer Menge von 0,001 bis 1 Gew.-%, vorzugsweise 0,001 bis 0,5 Gew.-% und besonders bevorzugt 0,001 bis 0,1 Gew.-%, bezogen auf die Menge an Feststoff in der Suspension, in dem flüssigen Medium verwendet. Beispiele für geeignete Entschäumer sind Paraffin, Siliconöle, Alkylpolysiloxane, höhere Alkohole, Propylenglykol, Ethylenoxid-Propylenoxid-Addukte und insbesondere Alkylpolyalkylenglykolether.

Es ist möglich, dass Komponenten des flüssigen Mediums mehrere Funktionen ausüben und z.B. sowohl Dispergiermittel als auch Bindemittel oder sowohl Dispergiermittel als auch Mittel zur Einstellung des pH-Wertes und Stabilisierungsmittel sind. Aufgrund des geringen Anteils an organischen Komponenten können diese auch innerhalb kurzer Zeit aus dem Rohling ausgebrannt werden. Dieser Vorgang wird üblicherweise auch als Entbindern bezeichnet.

Es ist weiter bevorzugt, dass die Suspension eine Viskosität von 5 mPas bis 500 mPas, bevorzugt 5 mPas bis 400 mPas und besonders bevorzugt 5 bis 300 mPas aufweist. Die Viskosität wurde mit einem Rotationsviskosimeter mit Platten-Kegel-System, Durchmesser 50 mm und Winkel 1° (MCR302-Modular Compact Rheometer, Firma Anton Paar GmbH), bei einer Scherrate im Bereich von 0,1 bis 1000 s$^{-1}$ und einer Temperatur von 25°C gemessen.

Zur Erzeugung der Suspension wird typischerweise das Zirkonoxid in Pulverform mit dem flüssigen Medium innig vermischt. Dabei können auch Gemische von zum Beispiel unterschiedlich gefärbtem Zirkonoxid verwendet werden. Bei dieser Vermischung werden üblicherweise auch vorhandene Agglomerate aufgebrochen und es kann auch ein Mahlen des eingesetzten Zirkonoxids erfolgen, um die gewünschte Teilchengröße zu erzeugen. Das Vermischen von Zirkonoxid und flüssigem Medium kann daher zum Beispiel vorteilhaft in Rührwerkskugelmühlen durchgeführt werden.

Die Suspension wird dann in Schritt (a) des erfindungsgemäßen Verfahrens in eine Form eingebracht, die über Poren verfügt. Das Einbringen erfolgt dabei üblicherweise durch Eingießen. Bei den Poren handelt es sich um Ausnehmungen, durch die das flüssige Medium aus der Suspension in Schritt (b) zumindest teilweise entfernt werden kann, sodass sich in der Form Zirkonoxid-Teilchen abscheiden und schließlich den Rohling bilden können. Die Geometrie der Form entspricht damit der Geometrie des gewünschten Rohlings. Die Form kann daher beispielsweise auch bereits eine einstückig mit dem Rohling ausgebildete Haltevorrichtung, wie einen Haltezapfen, aufweisen, was dessen späteres Anbringen zum Beispiel durch Ankleben überflüssig macht.

In einer bevorzugten Ausführungsform des erfindungsgemäßen Verfahrens weist daher der in Schritt (c) gebildete Rohling eine Haltevorrichtung auf.

Typischerweise wird im Wesentlichen das gesamte flüssige Medium über die Poren entfernt. Es ist allerdings auch möglich, dass verbleibende nicht über die Poren entfernte Flüssigkeit aus der Form abgegossen oder abgesaugt wird. Das wird üblicherweise dann der Fall sein, wenn sich bereits eine ausreichend dicke Schicht von Zirkonoxidteilchen abgeschieden hat.

Zur Erzeugung von Rohlingen, die Bereiche mit unterschiedlicher Färbung und/oder Transluzenz aufweisen, können nacheinander Suspensionen von unterschiedlich zusammengesetzten Zirkonoxid-Pulvern in die Form eingebracht und Schichten von unterschiedlichen Zirkonoxid-Teilchen jeweils gewünschter Dicke abgeschieden werden.

In einer bevorzugten Ausführungsform des erfindungsgemäßen Verfahrens werden daher in Schritt (a) nacheinander Suspensionen von Zirkonoxidpulvern mit unterschiedlicher Zusammensetzung und insbesondere mit unterschiedlicher Färbung und/oder Transluzenz in die Form eingebracht. Dabei werden typischerweise Maßnahmen ergriffen, dass sich die Suspensionen mit unterschiedlicher Zusammensetzung nicht durchmischen. Das kann z.B. dadurch erreicht werden, dass die aus einer Suspension abgeschiedene Schicht zunächst getrocknet und damit verfestigt wird, bevor eine weitere Suspension mit anderer Zusammensetzung in die Form eingebracht wird.

Bei der Form kann es sich beispielsweise um eine der üblicherweise für Schlickerguss- oder Druckgussverfahren eingesetzte Formen handeln. Dies sind insbesondere Formen mit einer Wandung aus Gips, durch die aufgrund der Kapillarwirkung der Gipsporen der Suspension Wasser entzogen werden kann. Es sind aber auch Formen aus Kunststoff, Keramik oder Metall einsetzbar, die bereits über Poren verfügen oder bei denen Poren vorgesehen werden, indem sie z.B. mit Filterelementen, wie Membran-, Papier- und Sinterfiltern, versehen werden.

Die eingesetzte Form ist insbesondere mehrteilig, um die einfache Entfernung des gebildeten Rohlings aus der Form zu erleichtern. In einer besonders bevorzugten Ausführungsform weist die Form Anschlüsse auf, über die auf die eingebrachte Suspension Druck, zum Beispiel mittels Druckluft, ausgeübt und/oder ein Unterdruck an die Poren angelegt werden kann. Beide Maßnahmen dienen dazu, die Entfernung des flüssigen Mediums aus der Form zu beschleunigen und damit das Verfahren abzukürzen. Mit ihrer Hilfe ist eine sehr schnelle und damit ökonomische Herstellung von Zirkonoxid-Rohlingen möglich, was insbesondere bei einer Fertigung in industriellem Maßstab von besonderem Vorteil ist.

Der mittels des erfindungsgemäßen Verfahrens gebildete Rohling kann eine beliebige gewünschte Form haben. Dabei wird insbesondere eine Form gewählt, die eine einfache maschinelle Bearbeitung des Rohlings in üblichen dentalen Schleif- und Fräsvorrichtungen gestattet. Der Rohling liegt bevorzugt als Block, Block mit einer Schnittstelle, wie z.B. einem Loch als Implantatschnittstelle, Scheibe oder zahnähnliche Vorform (Preform) vor. Dabei ist es bevorzugt, dass der Rohling auch eine Haltevorrichtung, wie einen Haltezapfen, aufweist, welche einstückig mit dem Rohling ausgebildet ist. Denn dadurch wird ein bisher übliches späteres Anbringen eines Halters durch Ankleben überflüssig gemacht. Die Haltevorrichtung dient der Aufnahme des Rohlings in einer Bearbeitungsvorrichtung, wie einer CAM-Maschine.

Nach dem Entfernen aus der Form in Schritt (c) wird der gebildete Rohling üblicherweise getrocknet. Die Trocknung erfolgt insbesondere bei einer Temperatur von 20 bis 60°C, bevorzugt 20 bis 50°C und besonders bevorzugt 20 bis 40°C. Die Dauer der Trocknung beträgt insbesondere 0,1 bis 24 h, bevorzugt 0,5 bis 12 h und besonders bevorzugt 1 bis 6 h. Die Trocknung kann z.B. in einem Klimaschrank oder durch Mikrowellentrocknung erfolgen.

Der nach Abschluss von Schritt (c) und gegebenenfalls anschließender Trocknung erhaltene Rohling liegt im sogenannten Grünzustand vor und er wird daher auch als Grünling bezeichnet. Überraschenderweise hat der Rohling in diesem Zustand eine sehr hohe Dichte und es ist bevorzugt, dass er eine Dichte von 3,3 bis 4,0 g/cm$^3$, insbesondere 3,35 bis 3,9 g/cm$^3$ und bevorzugt 3,4 bis 3,9 g/cm$^3$ hat. Die Dichte wurde mittels Quecksilberporosimetrie nach ISO 15901-1:2016 bestimmt.

In einer weiteren bevorzugten Ausführungsform hat dieser im Grünzustand vorliegende Rohling ein Porenvolumen von 0,08 bis 0,14 cm$^3$/g, insbesondere 0,08 bis 0,12 cm$^3$/g und besonders bevorzugt 0,08 bis 0,10 cm$^3$/g. Das Porenvolumen wurde mittels Quecksilberporosimetrie nach ISO 15901-1:2016 bestimmt.

In einer anderen bevorzugten Ausführungsform hat dieser im Grünzustand vorliegende Rohling einen Porendurchmesser von 0,02 bis 0,12 $\mu$m, insbesondere 0,03 bis 0,10 $\mu$m und besonders bevorzugt 0,04 bis 0,08 $\mu$m, gemessen als $D_{50}$-Wert bezogen auf das Volumen der Teilchen. Der Porendurchmesser wurde mittels Quecksilberporosimetrie nach ISO 15901-1:2016 bestimmt.

Der Rohling kann bereits in diesem Grünzustand, gegebenenfalls nach vorherigem Entfernen organischer Komponenten, maschinell bearbeitet werden, um ihm die Form der gewünschten dentalen Restauration zu verleihen.

Das Entfernen von organischen Komponenten wird auch als Entbindern bezeichnet und erfolgt insbesondere durch Wärmebehandlung bei einer Temperatur von etwa 600°C. Dabei wird üblicherweise eine Aufheizgeschwindigkeit von 0,125°K/min bis 10 K/min, bevorzugt 0,250 K/min bis 5 K/min und besonders bevorzugt 0,5 K/min bis 2 K/min ausgehend von Raumtemperatur auf etwa 600°C gewählt.

Es ist allerdings bevorzugt, dass der im Grünzustand befindliche Rohling auch vorgesintert wird. Durch die Vorsinterung werden die Festigkeit und Härte des Rohlings erhöht. Die Vorsinterung erfolgt dabei insbesondere bei einer Temperatur in Bereich von 600 bis 1100°C, bevorzugt bei 700 bis 1050°C und besonders bevorzugt bei 800 bis 1000°C für eine Dauer von insbesondere 5 min bis 24 h, bevorzugt 10 min bis 12 h und besonders bevorzugt 30 min bis 6 h.

Es ist bevorzugt, dass der vorgesinterte Rohling eine Dichte von 3,4 bis 4,0 g/cm$^3$, insbesondere 3,5 bis 4,0 g/cm$^3$ und besonders bevorzugt 3,6 bis 3,9 g/cm$^3$ hat.

Gemäß einer weiteren bevorzugten Ausführungsform hat der vorgesinterte Rohling ein Porenvolumen von 0,08 bis 0,14 cm$^3$/g, insbesondere 0,08 bis 0,12 cm$^3$/g und bevorzugt 0,09 bis 0,11 cm$^3$/g. Das Porenvolumen wurde mittels Quecksilberporosimetrie nach ISO 15901-1:2016 bestimmt.

In einer weiteren bevorzugten Ausführungsform hat der vorgesinterte Rohling einen maximalen Porendurchmesser von weniger als 0,15 μm, bevorzugt weniger als 0,12 μm und besonders bevorzugt weniger als 0,08 μm. Der maximale Porendurchmesser wurde mittels Quecksilberporosimetrie nach ISO 15901-1:2016 bestimmt.

Die Erfindung ist auch auf einen Zirkonoxid-Rohling gerichtet, der gemäß dem erfindungsgemäßen Verfahren erhältlich ist. Dieser Rohling verfügt offenbar über eine besondere Struktur im Vergleich zu konventionellen Rohlingen, da er nach gewünschter Formgebung innerhalb sehr kurzer Zeit zu einer dentalen Restauration mit sehr guten optischen Eigenschaften, wie Transluzenz, dichtgesintert werden kann.

Die Erfindung ist weiter auf einen Zirkonoxid-Rohling gerichtet, der vorgesintert ist und eine Dichte von 3,4 bis 4,0 $g/cm^3$, insbesondere 3,5 bis 4,0 $g/cm^3$ und bevorzugt 3,6 bis 3,9 $g/cm^3$ und/oder ein Porenvolumen von 0,08 bis 0,14 $cm^3/g$, insbesondere 0,08 bis 0,12 $cm^3/g$ und bevorzugt 0,09 bis 0,11 $cm^3/g$ und/oder einen maximalen Porendurchmesser von weniger als 0,15 μm, insbesondere weniger als 0,12 μm und bevorzugt weniger als 0,08 μm hat.

Die erfindungsgemäßen Rohlinge können in vorteilhafter Weise eingesetzt werden, um aus ihnen dentale Restaurationen zu fertigen. Dabei können Sie nach der Formgebung zu der gewünschten dentalen Restauration in nur sehr kurzer Zeit zu dentalen Restaurationen mit ausgezeichneten optischen Eigenschaften und hoher Festigkeit dichtgesintert werden, was eine sehr schnelle Versorgung eines Patienten mit einer dentalen Restauration ermöglicht.

Die erfindungsgemäßen Rohlinge zeichnen sich überdies dadurch aus, dass sie bei der Dichtsinterung eine nur geringe lineare Schwindung erfahren. Dadurch wird die Erzeugung von dentalen Restaurationen mit genau den gewünschten Dimensionen erleichtert und deren Passgenauigkeit verbessert.

Es ist bevorzugt, dass die erfindungsgemäßen Rohlinge eine lineare Schwindung von weniger als 18%, insbesondere weniger als 17% und besonders bevorzugt weniger als 16% haben. Die lineare Schwindung S ergibt sich aus der folgenden Formel und zu ihrer Bestimmung wurden Messungen mittels Netzsch-Dilatometer DIL402 Supreme in einem Temperaturbereich von 20°C bis 1550°C bei einer Aufheizgeschwindigkeit von 2 K/min an Prüfkörpern der Dimensionen Länge = 25mm ± 1mmm, Breite = 5mm ± 0.5mm und Höhe = 4mm ± 0.5mm durchgeführt.

$$\text{Lineare Schwindung: } S = \frac{L\ddot{a}nge\ roh - L\ddot{a}nge\ gesintert}{L\ddot{a}nge\ roh} * 100\ in\ \%$$

Aus der linearen Schwindung S kann die Volumenschwindung $S_{Vol}$ gemäß der folgenden Formel berechnet werden:

$$\text{Volumenschwindung: } S_{Vol} = \left[ 1 - \left( 1 - \frac{Lineare\ Schwindung}{100} \right)^3 \right] * 100\ in\ \%$$

Die Erfindung ist daher auch auf die Verwendung der erfindungsgemäßen Rohlinge zur Herstellung einer dentalen Restauration gerichtet. Bevorzugte Ausführungsformen dieser Verwendung werden im Folgenden im Zusammenhang mit der Beschreibung des erfindungsgemäßen Verfahrens zur Herstellung einer dentalen Restauration erläutert.

Das erfindungsgemäße Verfahren zur Herstellung einer dentalen Restauration zeichnet sich dadurch aus, dass einem erfindungsgemäßen Rohling die Form der dentalen Restauration gegeben und der Rohling bei einer Sintertemperatur von 1200 bis 1600°C, insbesondere 1300 bis 1550°C, bevorzugt 1350 bis 1500°C dichtgesintert wird.

Die Formgebung des Rohlings erfolgt dabei insbesondere durch maschinelle Bearbeitung, z.B. mittels computergesteuerter Schleif- und Fräsvorrichtungen, wie sie im Dentalbereich üblich sind.

Im Anschluss an die Formgebung kann gegebenenfalls auch noch Färbelösung auf den Rohling aufgebracht werden, um die gewünschte Einfärbung bei der schließlich erhaltenen dentalen Restauration zu erzielen.

Die anschließende Dichtsinterung führt dazu, dass der zur dentalen Restauration geformte Rohling eine Dichte von insbesondere mehr als 5,9 $g/cm^3$, bevorzugt mehr als 6,00 $g/cm^3$ und besonders bevorzugt mehr als 6,02 $g/cm^3$ hat.

Auch aufgrund dieser hohen Dichte verfügt die erhaltene dentale Restauration über ausgezeichnete mechanische Eigenschaften.

Es ist ein besonderer Vorteil, dass der gesamte Dichtsinterprozess mit dem Aufheizen von Raumtemperatur, dem Halten bei der maximalen Sintertemperatur und dem Abkühlen einen nur sehr kurzen Zeitraum in Anspruch nimmt und nach seinem Abschluss dennoch dentale Restaurationen mit der angestrebten hohen Transluzenz und sehr guten mechanischen Eigenschaften erhalten werden. Das erfindungsgemäße Verfahren ist damit konventionellen Verfahren überlegen, die einen sehr langen Zeitraum benötigen, um durch Sinterung eine dentale Restauration mit vergleichbarer Transluzenz zu erzeugen. Das erfindungsgemäße Verfahren vereint damit den Vorteil einer sehr kurzen Verfahrensdauer mit dem der sehr guten optischen und mechanischen Eigenschaften der erzeugten dentalen Restauration.

In einer bevorzugten Ausführungsform des erfindungsgemäßen Verfahrens beträgt der Zeitraum für das Aufheizen des geformten Rohlings von Raumtemperatur auf die Sintertemperatur für das Dichtsintern, das Halten bei der Sintertemperatur und das Abkühlen auf die Endtemperatur nicht mehr als 120 min, insbesondere nicht mehr als 60 min, bevorzugt nicht mehr als 40 min und besonders bevorzugt nicht mehr als 30 min. Dabei wird unter "Endtemperatur" eine Temperatur verstanden, bei der die Probe in die Hand genommen werden kann, und sie beträgt insbesondere 15 bis 80°C, bevorzugt 25 bis 60°C und besonders bevorzugt etwa 50°C. Unter "Raumtemperatur" wird eine Temperatur von insbesondere 15 bis 30°C, bevorzugt 20 bis 25°C und besonders bevorzugt etwa 25°C verstanden.

Die Aufheizgeschwindigkeit beträgt insbesondere mehr als 50 K/min, bevorzugt mehr als 100 K/min und besonders bevorzugt mehr als 200 K/min. Die Haltezeit beträgt insbesondere weniger als 30 min, bevorzugt weniger als 20 min und besonders bevorzugt weniger als 10 min. Die Abkühlgeschwindigkeit von der Sintertemperatur auf die Endtemperatur beträgt insbesondere mehr als 50 K/min, bevorzugt mehr als 100 K/min und besonders bevorzugt mehr als 150 K/min.

Durch die geringe lineare Schwingung, die der geformte erfindungsgemäße Rohling bei der Dichtsinterung erfährt, wird die Erzeugung von dentalen Restaurationen mit genau den gewünschten Dimensionen erleichtert und deren Passgenauigkeit verbessert.

In einer bevorzugten Ausführungsform wird der Rohling

    (a) einer ersten Wärmebehandlung unterworfen,
    (b) einer zweiten Wärmebehandlung unterworfen und
    (c) abgekühlt,

wobei die Wärmebehandlung in Schritt (a) bei niedrigerem Druck als die Wärmebehandlung in Schritt (b) erfolgt. Vorzugsweise wird der Rohling in Schritt (a) auf eine Temperatur aufgeheizt, die im Bereich von 1100 bis 1600°C, insbesondere im Bereich von 1200 bis 1500°C, bevorzugt im Bereich von 1250 bis 1450°C und weiter bevorzugt im Bereich von 1300 bis 1400°C liegt und am meisten bevorzugt etwa 1350°C beträgt. Dabei ist es weiter bevorzugt, dass die Wärmebehandlung in Schritt (a) bei einem Druck von weniger als 200 mbar, vorzugsweise weniger als 100 mbar und besonders bevorzugt weniger als 50 mbar und insbesondere bei einem Druck im Bereich von 0,1 bis 200 mbar, vorzugsweise 1 bis 150 mbar und am besonders bevorzugt 50 bis 100 mbar erfolgt. In Schritt (b) wird der Rohling vorzugsweise (b1) gegebenenfalls weiter aufgeheizt und (b2) bei einer, vorzugsweise konstanten, Temperatur im Bereich von 1100 bis 1700°C, insbesondere im Bereich von 1300 bis 1600°C und bevorzugt im Bereich von 1350 bis 1550°C und besonders bevorzugt bei einer Temperatur von etwa 1500°C gehalten und gesintert. Ebenfalls ist es bevorzugt, dass die Wärmebehandlung in Schritt (b) bei einem Druck von mehr als 500 mbar und insbesondere bei Umgebungsdruck erfolgt und insbesondere in einer sauerstoffhaltigen Atmosphäre wie Luft, mit Sauerstoff angereicherter Luft oder Sauerstoff erfolgt. Besonders bevorzugt wird die zum Aufheizen verwendete Heizkammer während Schritt (b) diskontinuierlich oder vorzugsweise kontinuierlich mit einer sauerstoffhaltigen Atmosphäre, vorzugsweise Luft, mit Sauerstoff angereicherter Luft oder Sauerstoff, durchströmt, insbesondere mit einer Strömungsgeschwindigkeit von 0,1 bis 50 l/min, bevorzugt 1 bis 20 l/min und besonders bevorzugt 4 bis 8 l/min. In einer besonders bevorzugten Ausführungsform wird der Rohling in Schritt (a) auf eine Temperatur aufgeheizt, die 0 bis 500 K, insbesondere 10 bis 250 K, bevorzugt 50 bis 200 K und besonders bevorzugt 100 bis 150 K unterhalb der Temperatur oder des Temperaturbereichs liegt, bei der/dem der Rohling in Schritt (b) gehalten wird.

Die nach der Dichtsinterung erhaltene dentale Restauration kann gegebenenfalls noch mit einer Verblendung versehen, poliert und/oder glasiert werden.

Bei der durch das erfindungsgemäße Verfahren hergestellten dentalen Restauration handelt es sich insbesondere um eine Brücke, ein Inlay, ein Onlay, eine Krone, ein Veneer, ein Implantat, eine Schale oder ein Abutment.

Die Erfindung wird im Folgenden anhand von Beispielen näher erläutert.

**Beispiele**

**Beispiel 1 - Suspension mit 76 Gew.-% Zirkonoxid**

In 194,4 g destilliertem Wasser wurden nacheinander 3,15 g eines Citronensäure- oder Citronensäuresalzhaltigen Dispergiermittels (Dolapix CE64, Firma Zschimmer & Schwarz) und 1,5 g Tetramethylammoniumhydroxid gelöst. Die Lösung hatte einen pH-Wert von 10-10,5.

Diese Lösung wurde in den Vorratsbehälter einer MicroCer-Rührwerkskugelmühle (Firma Netzsch) gegeben, deren Mahlraum sowie Rotor aus Zirkonoxid bestand. Der Mahlraum wurde mit 60 ml Zirkonoxid-Mahlperlen mit einem Durchmesser von 0.2-0.3 mm (Firma Tosoh) gefüllt. Bei einer Umdrehungsgeschwindigkeit des Rotors von 1500 U/min wurde die Lösung mit einer Schlauchpumpe (Schlauchinnendurchmesser 8 mm) kontinuierlich durch den Mahlraum gepumpt. Zu der Lösung im Vorratsbehälter wurden dann unter Rühren kontinuierlich 630 g Zirkonoxidpulver zugegeben, welches mit 3 Mol.-% $Y_2O_3$ teilstabilisiert war (TZ-PX-245 von TOSOH Corporation, Primärpartikelgrösse: 40 nm). Nach vollständiger Zugabe des Zirkonoxidpulvers wurde das erhaltene Gemisch für 45 min kontinuierlich durch den Mahlraum und wieder in den Vorratsbehälter mit einer Rate von ca. 40 l/h gepumpt. Die auf diese Weise hergestellte Suspension wurde in einen Kunststoffmessbecher überführt und mittels eines Magnetrührers sehr langsam gerührt, um eingeschlossene Luftblasen zu entfernen. Es wurde zusätzlich ein Tropfen eines Alkylpolyalkylenglykolethers als Entschäumer (Contraspum, Firma Zschimmer & Schwarz) zugegeben.

Die erhaltene Suspension hatte einen Gehalt an Zirkonoxid von 76 Gew.-%. Die Viskosität $\eta$ der Suspension betrug 7,25 mPas (bei einer Scherrate von 500 s$^{-1}$ und einer Temperatur von 25°C).

(A) Herstellung von Prüfkörpern

Es wurden Prüfkörper hergestellt, an denen verschiedene Eigenschaften bestimmt wurden. Zu deren Herstellung wurde die erhaltene Suspension in eine scheibenförmige Form gegossen, die aus einer Platte aus porösem Gips mit darauf befestigtem Kunststoffring bestand. Durch die Kapillarwirkung des porösen Gipses wurde der Suspension Wasser entzogen, und es bildete sich ein monolithischer Grünkörper aus. Der Grünkörper wurde in der Form belassen und für 24 h an der Luft getrocknet. Nach Abschluß der Trocknung hatte sich der Prüfkörper selbstständig von der Form gelöst.

Die jeweiligen Prüfkörper hatten folgende Eigenschaften:

- Lineare Schwindung: 15,43% (Prüfkörper: Länge = 25 mm $\pm$ 1 mm, Breite = 5 mm $\pm$ 0.5 mm und Höhe = 4 mm $\pm$ 0.5 mm)

- Dichte: 3,678 g/cm$^3$ (Prüfkörper: Länge = 5 mm $\pm$ 1 mm, Breite = 5 mm $\pm$ 1 mm und Höhe = 10 mm $\pm$ 1 mm)

(B) Herstellung von Blöcken für eine "chairside"-Anwendung

Zur Herstellung von Blöcken für eine "chairside"-Anwendung wurde eine Druckgussform verwendet. Diese Druckgussform bestand aus einem Aufsatz mit einem Anschluss für Druckluft (Überdruck 0.1 bis 100 bar), einem Mittelteil, das der gewünschten Form des Blockes entsprach, und einem Unterteil, das mit einem Membran-, Papier- und Sinterfilter versehen war und am Boden einen Anschluss zum Anlegen eines Vakuums aufwies. Der Membranfilter hatte eine Porengrösse von 200 nm.

Die Suspension wurde in die Druckgussform eingefüllt. Danach wurden über den Aufsatz Druckluft mit einem Druck von 6 bar zugegeben und über das Unterteil Vakuum von etwa 0,05 bar angelegt. Nach 4 h wurde die Zugabe von Druckluft sowie das Anlegen von Vakuum beendet. Die Druckgussform wurde auseinandergebaut und der erhaltene Block wurde an der Luft bei 20°C für 24 h getrocknet.

Die auf diese Weise hergestellten Blöcke wurden bei 950°C für 2 h vorgesintert. Die Aufheizgeschwindigkeit betrug 0,250 K/min. Anschließend wurden die Blöcke innerhalb von 24 h auf Raumtemperatur abgekühlt. An den Blöcken wurde die Härte mittels Vickers-Methode nach ISO 14705:2008 und EN ISO 6507-1:2005 bei einer Last von 2,5 kg gemessen. Die Härte betrug 992,95 N/mm$^2$.

Aus den vorgesinterten Blöcken wurde mittels einer CAD/CAM-Maschine (Zenotec® Select von Ivoclar Vivadent AG) eine Frontzahnkrone trocken gefräst, auf die zur Einfärbung mittels Pinsel Färbeflüssigkeit aufgebracht wurde. Nach dem Einfärben wurde die Krone im Sinterofen (Programat CS4, Programm 1, von Ivoclar Vivadent AG) getrocknet und dichtgesintert. Die Sinterung dauerte nur etwa 35 min, wie das nachstehende Temperaturprogramm zeigt.

| | |
|---|---|
| Ca. 25°C bis 900°C | (6,7 min) |
| 900°C bis 1460°C | (11,2 min) |
| 1460°C bis 1460°C | (5,0 min) |
| 1460°C bis 1200°C | (3,7 min) |
| 1200°C bis ca. 1000-950°C | (45-50 s) (Ofen öffnet) |
| 950°C-1000°C bis ca. 50°C | (7 min) |

**Beispiel 2 - Suspension mit 83 Gew.-% Zirkonoxid**

Zur Herstellung einer Suspension mit 83 Gew.-% Zirkonoxid und deren Verarbeitung zu Prüfkörpern (A) und Blöcken für die "chairside"-Anwendung (B) wurde Beispiel 1 mit der Abweichung wiederholt, dass die Lösung 164,5 g destilliertes Wasser, 4,05 g Citronensäure- oder Citronensäuresalz-haltiges Dispergiermittel (Dolapix CE64, Firma Zschimmer & Schwarz) und 1,5 g Tetramethylammoniumhydroxid enthielt und 810 g Zirkonoxidpulver zugegeben wurden, welches mit 5 Mol.-% Y$_2$O$_3$ teilstabilisiert war (TZ-PX-430 von TOSOH Corporation, Primärpartikelgrösse: 90 nm) und die Vorsinterung bei 900°C für 2 h durchgeführt wurde, wobei die Aufheizgeschwindigkeit ebenfalls 0,250 K/min betrug.

Die Viskosität η der Suspension betrug 7,0 mPas (bei einer Scherrate von 1000 s$^{-1}$ und einer Temperatur von 25°C).

Die gemäß (A) erhaltenen jeweiligen Prüfkörper hatten folgende Eigenschaften:

- Lineare Schwindung: 14,43% (Prüfkörper: Länge = 25mm $\pm$ 1mm, Breite = 5mm $\pm$ 0.5mm und Höhe = 4mm $\pm$ 0.5mm)

- Dichte: 3,791 g/cm$^3$ (Prüfkörper: Länge = 5mm $\pm$ 1mm, Breite = 5mm $\pm$ 1mm und Höhe = 10mm $\pm$ 1mm)

An den gemäß (B) erhaltenen vorgesinterten Blöcken wurde die Härte mittels Vicker's-Methode nach ISO 14705:2008 und EN ISO 6507-1:2005 bei einer Last von 2,5 kg gemessen. Die Härte betrug 496,91 N/mm$^2$.

Aus den vorgesinterten Blöcken wurde mittels einer CAD/CAM-Maschine (Zenotec® Select von Ivoclar Vivadent AG) eine Frontzahnkrone trocken gefräst, auf die zur Einfärbung mittels Pinsel Färbeflüssigkeit aufgebracht wurde. Nach dem Einfärben wurde die Krone im Sinterofen (Programat CS4, Programm 1, von Ivoclar Vivadent AG) getrocknet und dichtgesintert. Die Sinterung dauerte nur 34 min, wie das nachstehende Temperaturprogramm zeigt.

| | |
|---|---|
| Ca. 25°C bis 900°C | (6,7 min) |
| 900°C bis 1460°C | (11,2 min) |
| 1460°C bis 1460°C | (5, 0 min) |
| 1460°C bis 1200°C | (3,7 min) |
| 1200°C bis ca. 1000-950°C | (45-50 s) (Ofen öffnet) |
| 950°C-1000°C bis ca. 50°C | (7 min) |

### Beispiel 3 - Suspension mit 80 Gew.-% Zirkonoxid

Zur Herstellung einer Suspension mit 80 Gew.-% Zirkonoxid und deren Verarbeitung zu Prüfkörpern (A) wurde Beispiel 1 mit der Abweichung wiederholt, dass die Lösung 179,5 g destilliertes Wasser, 3,6 g Citronensäure- oder Citronensäuresalz-haltiges Dispergiermittel (Dolapix CE64, Firma Zschimmer & Schwarz) und 1,5 g Tetramethylammoniumhydroxid enthielt und 720 g Zirkonoxidpulver zugegeben wurden, welches mit 4,25 Mol.-% $Y_2O_3$ teilstabilisiert war (TZ-PX-551 von TOSOH Corporation, Primärpartikelgrösse: 90 nm).

Die Viskosität η der Suspension betrug 14,6 mPas (bei einer Scherrate von 500 s$^{-1}$ und einer Temperatur von 25°C).

Die nach (A) erhaltenen Prüfkörper hatten folgende Eigenschaften:

- Lineare Schwindung: 14,59%
- Dichte: 3,780 g/cm$^3$.

Die nach (A) erhaltenen Prüfkörper wurden bei 1000°C für 2 h vorgesintert. Die Aufheizgeschwindigkeit betrug 0,250 K/min. Anschließend wurden die Prüfkörper innerhalb von 24 h auf Raumtemperatur abgekühlt. Die vorgesinterten Prüfkörper hatten eine lineare Schwindung von 13,88 %.

### Beispiel 4 - Suspension mit 76 Gew.-% Zirkonoxid

Zur Herstellung einer Suspension mit 76 Gew.-% Zirkonoxid und deren Verarbeitung zu Blöcken für die "chairside"-Anwendung (B) wurde Beispiel 1 mit der Abweichung wiederholt, dass zu der Lösung 630 g Zirkonoxidpulver zugegeben wurden, welches mit 3 Mol.-% $Y_2O_3$ teilstabilisiert war (AuerDent 3Y-5A A2 von Treibacher Industrie AG, Primärpartikelgrösse: 40 nm) und die Vorsinterung bei 820°C für 2 h durchgeführt wurde, wobei die Aufheizgeschwindigkeit ebenfalls 0,250 K/min betrug.

An den nach (B) erhaltenen vorgesinterten Blöcken wurde die Härte mittels Vickers-Methode nach ISO 14705:2008 und EN ISO 6507-1:2005 bei einer Last von 2.5 kg gemessen. Die Härte betrug 440,38 N/mm$^2$.

Weiter wurde von den vorgesinterten Blöcken eine Scheibe abgesägt und nach dem folgenden Temperaturprogramm im Sinterofen (Programat CS4 von Ivoclar Vivadent AG) innerhalb von nur 34 min dichtgesintert:

| | |
|---|---|
| Ca. 25°C bis 900°C | (6,7 min) |

(fortgesetzt)

| | |
|---|---|
| 900°C bis 1460°C | (11,2 min) |
| 1460°C bis 1460°C | (5, 0 min) |
| 1460°C bis 1200°C | (3,7 min) |
| 1200°C bis ca. 1000-950°C | (45-50 s)(Ofen öffnet) |
| 950°C-1000°C bis ca. 50°C | (7 min) |

Die dichtgesinterten Scheiben hatten einen Kontrastwert CR von 89,16. Der Kontrastwert wurde gemäß BS 5612 (Britischer Standard) unter Verwendung eines Spektralcolorimeters (Minolta CM-3700d) bestimmt. Er ist ein Maß für die Opazität eines Materials, wobei ein Kontrastwert von 100 einem vollständig opaken Material und eine Kontrastwert von 0 einem vollständig transluzentem Material entspricht.

Der gemessene Wert von 89,16 zeigt die hohe Transluzenz der erfindungsgemäß hergestellten Proben und dieser ist bei konventionell erzeugten Rohlingen nur bei sehr langen Sinterdauern von mehr als 2 Stunden erzielbar.

**Beispiel 5 - Suspension mit 83 Gew.-% Zirkonoxid**

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- oder Citronensäuresalz-haltiges Dispergiermittel (Dolapix CE64, Firma Zschimmer & Schwarz) und 1,5 g Tetramethylammoniumhydroxid enthielt und 810 g Zirkonoxidpulver zugegeben wurden, welches mit 3 Mol.-% $Y_2O_3$ teilstabilisiert war (TZ-PX-245 von TOSOH Corporation, Primärpartikelgrösse: 40 nm).

Die nach (A) erhaltenen Prüfkörper wurden bei 500°C entbindert und hatten dann folgende Eigenschaften:

- Porenvolumen: 0,11391 $cm^3$/g
- Porenradius: 0,0190 $\mu$m
- Dichte: 3,5617 g/$cm^3$ (Prüfkörper: Länge = 5mm $\pm$ 1mm, Breite = 5mm $\pm$ 1mm und Höhe = 10mm $\pm$ 1mm)

Anschliessend wurden die entbinderten Prüfkörper bei 1050°C für 2 h vorgesintert. Die erhaltenen vorgesinterten Rohlinge hatten dann folgende Eigenschaften:

- Porenvolumen: 0,09931 $cm^3$/g
- Porenradius: 0,0242 $\mu$m
- Dichte: 3,8866 g/$cm^3$ (Prüfkörper: Länge = 5mm $\pm$ 1mm, Breite = 5mm $\pm$ 1mm und Höhe = 10mm $\pm$ 1mm)

Entbinderte Prüfkörper wurden ebenfalls bei 1000°C für 2 h vorgesintert. Die erhaltenen vorgesinterten Rohlinge wurden nach dem folgenden Temperaturprogramm im Sinterofen (Cerec Speedfire von Dentsply Sirona) innerhalb von nur 20 min dichtgesintert:

| | |
|---|---|
| Ca. 25°C bis 500°C | (88 s) |
| 500°C bis 1580°C | (190 s) |
| 1580°C bis 1580°C | (360 s) |
| 1580°C bis 950°C | (120 s) |
| 950°C bis ca. 50°C | (420 s) |

**Beispiel 6 - Suspension mit 83 Gew.-% Zirkonoxid + Vakuum**

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 4,05 g Citronensäure- oder Citronensäuresalz-haltiges Dispergiermittel (Dolapix CE64, Firma Zschimmer & Schwarz) und 1,5 g Tetramethylammoniumhydroxid enthielt und 810 g Zirkonoxidpulver zugegeben wurden, welches mit 3 Mol.-% $Y_2O_3$ teilstabilisiert war (TZ-PX-245 von TOSOH Corporation, Primärpartikelgrösse: 40 nm).

Die nach (A) erhaltenen Prüfkörper wurden entbindert und vorgesintert, indem sie auf eine Temperatur von 1000°C mit einer Geschwindigkeit von 0,25 K/min aufgeheizt und bei dieser Temperatur für 2 h gehalten wurden.

Die erhaltenen vorgesinterten Rohlinge wurden nach dem folgenden Temperaturprogramm im Sinterofen mit $MoSi_2$-Heizelementen innerhalb von nur maximal 36 min dichtgesintert, wobei die ersten beiden Schritte unter Vakuum mit einem Druck im Bereich von etwa 50 bis 100 mbar und nach Zuströmen von Luft die weiteren Schritte unter Umgebungsatmosphäre und kontinuierlichem Spülen mit Luft durchgeführt wurden.

| | | |
|---|---|---|
| Ca. 25°C bis 950°C | (7,1 min) | unter Vakuum |
| 950°C bis 1450°C | (10 min) | unter Vakuum |
| 1450°C bis 1500°C | (1 min) | Kontinuierliches Durchströmen mit Luft bei Umgebungsatmosphäre |
| 1500°C bis 1500°C | (5 min) | Kontinuierliches Durchströmen mit Luft bei Umgebungsatmosphäre |
| 1500°C bis 1100°C | (2,9 min) | Umgebungsatmosphäre |
| 1100°C bis ca. 50°C | (5-10 min) | Umgebungsatmosphäre |

Für die Untersuchungen der optischen Eigenschaften wurden die dichtgesinterten Prüfkörper auf eine Dicke von 2 mm $\pm$ 0.02 mm und einem Durchmesser von 18 mm $\pm$ 0.5 mm planparallel geschliffen (20$\mu$m-Diamantscheibe) und anschließend poliert (SiC-Papier 1000 er Körnung).

Die dichtgesinterten Scheiben hatten einen Kontrastwert CR von nur 78,95%. Sie zeigten daher eine sehr hohe Transluzenz und den günstigen Einfluss des Einsatzes von Vakuum und des anschließenden Atmosphärentausches bei der Dichtsinterung.

### Beispiel 7 - Suspension mit 83 Gew.-% Zirkonoxid

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- oder Citronensäuresalz-haltiges Dispergiermittel (Dolapix CE64, Firma Zschimmer & Schwarz) und 2,0 g Tetramethylammoniumhydroxid enthielt und 810 g Zirkonoxidpulver zugegeben wurden, welches mit 4,25 Mol-% $Y_2O_3$ teilstabilisiert war (TZ-PX-551 von TOSOH Corporation, Primärpartikelgrösse: 90 nm).

Die nach (A) erhaltenen Prüfkörper wurden bei 500°C entbindert und hatten dann folgende Eigenschaften:

- Porenvolumen: 0,11087 cm$^3$/g
- Porenradius: 0,0248 $\mu$m
- Dichte: 3,5474 g/cm$^3$ (Prüfkörper: Länge = 5mm $\pm$ 1mm, Breite = 5mm $\pm$ 1mm und Höhe = 10mm $\pm$ 1mm)

Anschliessend wurden die entbinderten Prüfkörper bei 1050°C für 2 h vorgesintert. Die erhaltenen vorgesinterten Rohlinge hatten dann folgende Eigenschaften:

- Porenvolumen: 0,10282 cm$^3$/g
- Porenradius: 0,0315 $\mu$m
- Dichte: 3,6838 g/cm$^3$ (Prüfkörper: Länge = 5mm $\pm$ 1mm, Breite = 5mm $\pm$ 1mm und Höhe = 10mm $\pm$ 1mm)

### Beispiel 8 - Suspension mit 83 Gew.-% Zirkonoxid

Zur Herstellung einer Suspension mit 83 Gew.-% Zirkonoxid und deren Verarbeitung zu Prüfkörpern (A) wurde Beispiel 1 mit der Abweichung wiederholt, dass die Lösung 164,5 g destilliertes Wasser, 3,15 g Citronensäure- oder Citronensäuresalz-haltiges Dispergiermittel (Dolapix CE64, Firma Zschimmer & Schwarz) und 2,0 g Tetramethylammoniumhydroxid enthielt und 810 g Zirkonoxidpulver zugegeben wurden, welches mit 5,0 Mol-% $Y_2O_3$ teilstabilisiert war (TZ-PX-430 von TOSOH Corporation, Primärpartikelgrösse: 90 nm).

Die nach (A) erhaltenen Prüfkörper wurden bei 500°C entbindert und hatten dann folgende Eigenschaften:

- Porenvolumen: 0,10559 cm$^3$/g
- Porenradius: 0,0253 $\mu$m
- Dichte: 3,7831 g/cm$^3$ (Prüfkörper: Länge = 5 mm $\pm$ 1 mm, Breite = 5 mm $\pm$ 1 mm und Höhe = 10 mm $\pm$ 1 mm)

Anschliessend wurden die entbinderten Prüfkörper bei 1050°C für 2 h vorgesintert. Die erhaltenen vorgesinterten Rohlinge hatten dann folgende Eigenschaften:

- Porenvolumen: 0,10311 cm$^3$/g
- Mittlerer Porenradius: 0,0318 $\mu$m
- Dichte: 3,9083 g/cm$^3$ (Prüfkörper: Länge = 5 mm $\pm$ 1 mm, Breite = 5 mm $\pm$ 1 mm und Höhe = 10 mm $\pm$ 1 mm)

**Patentansprüche**

1. Verfahren zur Herstellung eines Zirkonoxid-Rohlings, bei dem

   (a) eine Suspension von Zirkonoxid in einem flüssigen Medium in eine Poren aufweisende Form eingebracht,
   (b) das flüssige Medium zumindest teilweise über die Poren entfernt und
   (c) der gebildete Rohling aus der Form entfernt wird,

   wobei die Suspension einen Gehalt an Zirkonoxid von 68 bis 88 Gew.-%, insbesondere 70 bis 86 Gew.-% und bevorzugt 75 bis 85 Gew.-% aufweist.

2. Verfahren nach Anspruch 1, bei dem das Zirkonoxid in der Suspension eine Teilchengröße von 50 bis 250 nm, insbesondere 60 bis 250 nm und bevorzugt 80 bis 250 nm, gemessen als $d_{50}$-Wert und bezogen auf das Volumen der Teilchen, aufweist.

3. Verfahren nach Anspruch 1 oder 2, bei dem das Zirkonoxid in der Suspension eine Primärpartikelgröße von 30 bis 100 nm hat.

4. Verfahren nach einem der Ansprüche 1 bis 3, bei dem das Zirkonoxid mit $Y_2O_3$, $La_2O_3$, $CeO_2$, MgO und/oder CaO stabilisiert ist und insbesondere mit 2 bis 14 Mol-%, bevorzugt 2 bis 10 Mol-% und besonders bevorzugt 2 bis 8 Mol-% dieser Oxide, bezogen auf den Gehalt an Zirkonoxid, stabilisiert ist.

5. Verfahren nach einem der Ansprüche 1 bis 4, bei dem das flüssige Medium Wasser enthält.

6. Verfahren nach einem der Ansprüche 1 bis 5, bei dem das flüssige Medium organische Komponenten in einer Menge von nicht mehr als 5 Gew.-%, insbesondere nicht mehr als 3 Gew.-%, bevorzugt nicht mehr als 2 Gew.-% und besonders bevorzugt nicht mehr als 1 Gew.-%, bezogen auf die Menge an Feststoff in der Suspension, oder in einer Menge von 0,05 bis 5 Gew.-%, insbesondere 0,1 bis 3 Gew.-%, bevorzugt 0,1 bis 2 Gew.-% und besonders bevorzugt 0,1 bis 1 Gew.-%, bezogen auf die Menge an Feststoff in der Suspension, enthält.

7. Verfahren nach einem der Ansprüche 1 bis 6, bei dem das flüssige Medium mindestens eine Verbindung ausgewählt aus Aminoalkohol, Glykol, Carbonsäure und Carbonsäuresalz und bevorzugt mindestens eine Verbindung ausgewählt aus Ethanolamin, Ethylenglykol, Dipropylenglykol, Citronensäure und Citronensäuresalz enthält.

8. Verfahren nach einem der Ansprüche 1 bis 7, bei dem die Suspension eine Viskosität von 5 bis 500 mPas, insbesondere 5 bis 400 mPas und bevorzugt 5 bis 300 mPas, gemessen bei einer Scherrate von 0,1 bis 1000 s$^{-1}$ und einer Temperatur von 25°C, hat.

9. Verfahren nach einem der Ansprüche 1 bis 8, bei dem die Suspension ein Gemisch von Zirkonoxidpulvern mit unterschiedlicher Zusammensetzung und insbesondere mit unterschiedlicher Färbung und/oder Transluzenz enthält.

10. Verfahren nach einem der Ansprüche 1 bis 9, bei dem in Schritt (a) nacheinander Suspensionen von Zirkonoxidpulvern mit unterschiedlicher Zusammensetzung und insbesondere mit unterschiedlicher Färbung und/oder Transluzenz in die Form eingebracht werden.

11. Verfahren nach einem der Ansprüche 1 bis 10, bei dem der Rohling ein Block, ein Block mit einer Schnittstelle, eine Scheibe oder eine zahnähnliche Vorform ist.

12. Verfahren nach einem der Ansprüche 1 bis 11, bei dem der in Schritt (c) gebildete Rohling eine Haltevorrichtung aufweist, die insbesondere einstückig mit dem Rohling ausgebildet ist.

13. Verfahren nach einem der Ansprüche 1 bis 12, bei dem der in Schritt (c) gebildete Rohling getrocknet und insbesondere bei einer Temperatur von 20 bis 60°C, bevorzugt 20 bis 50°C und besonders bevorzugt 20 bis 40°C getrocknet wird.

14. Verfahren nach einem der Ansprüche 1 bis 13, bei dem der Rohling eine Dichte von 3,3 bis 4,0 g/cm$^3$, insbesondere 3,35 bis 3,9 g/cm$^3$ und bevorzugt 3,4 bis 3,9 g/cm$^3$

und/oder

ein Porenvolumen von 0,08 bis 0,14 cm$^3$/g, insbesondere 0,08 bis 0,12 cm$^3$/g und bevorzugt 0,08 bis 0,10 cm$^3$/g

und/oder

einen Porendurchmesser von 0,02 bis 0,12 $\mu$m, insbesondere 0,03 bis 0,10 $\mu$m und bevorzugt 0,04 bis 0,08 $\mu$m, gemessen als D$_{50}$-Wert bezogen auf das Volumen der Teilchen, hat.

15. Verfahren nach einem der Ansprüche 1 bis 14, bei dem der Rohling vorgesintert wird und insbesondere bei einer Temperatur in Bereich von 600 bis 1100°C, bevorzugt 700 bis 1050°C und besonders bevorzugt 800 bis 1000°C vorgesintert wird.

16. Verfahren nach Anspruch 15, bei dem der vorgesinterte Rohling eine Dichte von 3,4 bis 4,0 g/cm$^3$, insbesondere 3,5 bis 4,0 g/cm$^3$ und bevorzugt 3,6 bis 3,9 g/cm$^3$

und/oder

ein Porenvolumen von 0,08 bis 0,14 cm$^3$/g, insbesondere 0,08 bis 0,12 cm$^3$/g und bevorzugt 0,09 bis 0,11 cm$^3$/g

und/oder

einen maximalen Porendurchmesser von weniger als 0,15 $\mu$m, insbesondere weniger als 0,12 $\mu$m und bevorzugt weniger als 0,08 $\mu$m hat.

17. Zirkonoxid-Rohling, der gemäß dem Verfahren nach einem der Ansprüche 1 bis 16 erhältlich ist.

18. Zirkonoxid-Rohling, der vorgesintert ist und eine Dichte von 3,4 bis 4,0 g/cm$^3$, insbesondere 3,5 bis 4,0 g/cm$^3$ und bevorzugt 3,6 bis 3,9 g/cm$^3$

und/oder

ein Porenvolumen von 0,08 bis 0,14 cm$^3$/g, insbesondere 0,08 bis 0,12 cm$^3$/g und bevorzugt 0,09 bis 0,11 cm$^3$/g

und/oder

einen maximalen Porendurchmesser von weniger als 0,15 $\mu$m, insbesondere weniger als 0,12 $\mu$m und bevorzugt weniger als 0,08 $\mu$m hat.

19. Zirkonoxid-Rohling nach Anspruch 17 oder 18, der eine lineare Schwindung von weniger als 18 %, insbesondere weniger als 17 % und bevorzugt weniger als 16 % aufweist.

20. Verwendung des Rohlings nach einem der Ansprüche 17 bis 19 zur Herstellung einer dentalen Restauration.

21. Verfahren zur Herstellung einer dentalen Restauration, bei dem dem Rohling nach einem der Ansprüche 17 bis 19 die Form der dentalen Restauration gegeben und der Rohling bei einer Sintertemperatur von 1200 bis 1600°C, insbesondere 1300 bis 1550°C und bevorzugt 1350 bis 1500°C dichtgesintert wird.

22. Verfahren nach Anspruch 21, bei dem der Zeitraum für das Aufheizen des Rohlings von Raumtemperatur auf die Sintertemperatur, das Halten bei der Sintertemperatur und das Abkühlen auf die Endtemperatur nicht mehr als 120 min, insbesondere nicht mehr als 60 min, bevorzugt nicht mehr als 40 min und besonders bevorzugt nicht mehr als 30 min beträgt.

23. Verfahren nach Anspruch 21 oder 22, bei dem der Rohling

(a) einer ersten Wärmebehandlung unterworfen wird,
(b) einer zweiten Wärmebehandlung unterworfen wird und
(c) abgekühlt wird,

wobei die Wärmebehandlung in Schritt (a) bei niedrigerem Druck als die Wärmebehandlung in Schritt (b) erfolgt.

24. Verfahren nach einem der Ansprüche 21 bis 23, bei dem die dentale Restauration eine Brücke, ein Inlay, ein Onlay, eine Krone, ein Implantat, ein Veneer, eine Schale oder ein Abutment ist.

**Europäisches Patentamt**
**European Patent Office**
**Office européen des brevets**

# EUROPÄISCHER RECHERCHENBERICHT

Nummer der Anmeldung

EP 18 20 9272

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | CN 107 696 235 A (ZHONGSHAN HUATONGDA CERAMICS TECH CO LTD) 16. Februar 2018 (2018-02-16) * das ganze Dokument * ----- | 1 | INV. A61K6/02 A61C13/00 |
| A,D | WO 2014/209626 A1 (IVOCLAR VIVADENT INC [US]) 31. Dezember 2014 (2014-12-31) * Ansprüche 1-65 * ----- | 1-17 | |
| A,D | WO 2018/049331 A1 (JENSEN IND INC [US]) 15. März 2018 (2018-03-15) * Ansprüche 1-17 * ----- | 1-17 | |
| X | WO 2008/148494 A1 (NOBEL BIOCARE SERVICES AG [CH]; ERIKSSON CECILIA [SE] ET AL.) 11. Dezember 2008 (2008-12-11) * Ansprüche 1-12 * * Seite 5, Zeile 4 - Zeile 5 * ----- | 18-24 | |
| X | EP 3 108 849 A1 (3M INNOVATIVE PROPERTIES CO [US]) 28. Dezember 2016 (2016-12-28) * Ansprüche 1-15 * * Absatz [0046] * ----- | 18-24 | RECHERCHIERTE SACHGEBIETE (IPC) A61K A61C |
| A,D | WO 2013/181018 A2 (GLIDEWELL JAMES R DENTAL CERAMICS INC [US]; CARDEN ROBIN A [US]) 5. Dezember 2013 (2013-12-05) * Ansprüche 1-20 * ----- | 18-24 | |

Der vorliegende Recherchenbericht wurde für alle Patentansprüche erstellt

| Recherchenort | Abschlußdatum der Recherche | Prüfer |
|---|---|---|
| Berlin | 23. August 2019 | Siatou, Evangelia |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

Europäisches
Patentamt

European
Patent Office

Office européen
des brevets

## GEBÜHRENPFLICHTIGE PATENTANSPRÜCHE

Die vorliegende europäische Patentanmeldung enthielt bei ihrer Einreichung Patentansprüche, für die eine Zahlung fällig war.

☐ Nur ein Teil der Anspruchsgebühren wurde innerhalb der vorgeschriebenen Frist entrichtet. Der vorliegende europäische Recherchenbericht wurde für jene Patentansprüche erstellt, für die keine Zahlung fällig war, sowie für die Patentansprüche, für die Anspruchsgebühren entrichtet wurden, nämlich Patentansprüche:

☐ Keine der Anspruchsgebühren wurde innerhalb der vorgeschriebenen Frist entrichtet. Der vorliegende europäische Recherchenbericht wurde für die Patentansprüche erstellt, für die keine Zahlung fällig war.

## MANGELNDE EINHEITLICHKEIT DER ERFINDUNG

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

Siehe Ergänzungsblatt B

☒ Alle weiteren Recherchengebühren wurden innerhalb der gesetzten Frist entrichtet. Der vorliegende europäische Recherchenbericht wurde für alle Patentansprüche erstellt.

☐ Da für alle recherchierbaren Ansprüche die Recherche ohne einen Arbeitsaufwand durchgeführt werden konnte, der eine zusätzliche Recherchengebühr gerechtfertigt hätte, hat die Recherchenabteilung nicht zur Zahlung einer solchen Gebühr aufgefordert.

☐ Nur ein Teil der weiteren Recherchengebühren wurde innerhalb der gesetzten Frist entrichtet. Der vorliegende europäische Recherchenbericht wurde für die Teile der Anmeldung erstellt, die sich auf Erfindungen beziehen, für die Recherchengebühren entrichtet worden sind, nämlich Patentansprüche:

☐ Keine der weiteren Recherchengebühren wurde innerhalb der gesetzten Frist entrichtet. Der vorliegende europäische Recherchenbericht wurde für die Teile der Anmeldung erstellt, die sich auf die zuerst in den Patentansprüchen erwähnte Erfindung beziehen, nämlich Patentansprüche:

☐ Der vorliegende ergänzende europäische Recherchenbericht wurde für die Teile der Anmeldung erstellt, die sich auf die zuerst in den Patentansprüchen erwähnte Erfindung beziehen (Regel 164 (1) EPÜ).

Europäisches
Patentamt

European
Patent Office

Office européen
des brevets

**MANGELNDE EINHEITLICHKEIT
DER ERFINDUNG
ERGÄNZUNGSBLATT B**

Nummer der Anmeldung

EP 18 20 9272

Nach Auffassung der Recherchenabteilung entspricht die vorliegende europäische Patentanmeldung nicht den Anforderungen an die Einheitlichkeit der Erfindung und enthält mehrere Erfindungen oder Gruppen von Erfindungen, nämlich:

1. Ansprüche: 1-17

   Verfahren zur Herstellung eines Zirkonoxid-Rohlings, wobei eine Suspension von Zirkonoxid in eine Poren aufweisende Form eingebracht, und der gebildete Rohling aus der Form entfernt wird.

   ---

2. Ansprüche: 18-24

   Zirkonoxid--Rohling, Verfahren zur Herstellung, und Verwendung zur Herstellung einer dentalen Restauration.

   ---

**EP 3 659 574 A1**

**ANHANG ZUM EUROPÄISCHEN RECHERCHENBERICHT
ÜBER DIE EUROPÄISCHE PATENTANMELDUNG NR.**

EP 18 20 9272

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

23-08-2019

| Im Recherchenbericht angeführtes Patentdokument | Datum der Veröffentlichung | Mitglied(er) der Patentfamilie | Datum der Veröffentlichung |
|---|---|---|---|
| CN 107696235 A | 16-02-2018 | KEINE | |
| WO 2014209626 A1 | 31-12-2014 | BR 112015032559 A2 | 25-07-2017 |
| | | CA 2913112 A1 | 31-12-2014 |
| | | CN 105338948 A | 17-02-2016 |
| | | EP 3013306 A1 | 04-05-2016 |
| | | JP 6416247 B2 | 31-10-2018 |
| | | JP 2016523286 A | 08-08-2016 |
| | | JP 2019005627 A | 17-01-2019 |
| | | KR 20160023766 A | 03-03-2016 |
| | | US 2016095798 A1 | 07-04-2016 |
| | | US 2018133112 A1 | 17-05-2018 |
| | | WO 2014209626 A1 | 31-12-2014 |
| WO 2018049331 A1 | 15-03-2018 | US 2018072628 A1 | 15-03-2018 |
| | | WO 2018049331 A1 | 15-03-2018 |
| WO 2008148494 A1 | 11-12-2008 | AT 507796 T | 15-05-2011 |
| | | CN 101715325 A | 26-05-2010 |
| | | EP 2000109 A1 | 10-12-2008 |
| | | ES 2366233 T3 | 18-10-2011 |
| | | JP 5380436 B2 | 08-01-2014 |
| | | JP 2010528730 A | 26-08-2010 |
| | | KR 20100018047 A | 16-02-2010 |
| | | US 2010323327 A1 | 23-12-2010 |
| | | US 2012326343 A1 | 27-12-2012 |
| | | WO 2008148494 A1 | 11-12-2008 |
| EP 3108849 A1 | 28-12-2016 | BR 112018071894 A2 | 05-02-2019 |
| | | CN 109069239 A | 21-12-2018 |
| | | EP 3108849 A1 | 28-12-2016 |
| | | JP 2019515030 A | 06-06-2019 |
| | | US 2019231494 A1 | 01-08-2019 |
| | | WO 2017189344 A1 | 02-11-2017 |
| WO 2013181018 A2 | 05-12-2013 | US 2013313738 A1 | 28-11-2013 |
| | | US 2016332918 A1 | 17-11-2016 |
| | | WO 2013181018 A2 | 05-12-2013 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2014209626 A
- WO 2009061410 A
- WO 2013181018 A

- EP 826642 A
- KR 101416654 B1
- WO 2018049331 A